# EUROPEAN PATENT SPECIFICATION

(11) **EP 1 684 794 B1**
(45) Date of publication and mention of the grant of the patent: **28.09.2016**
(21) Application number: 04793705.7
(22) Date of filing: 04.10.2004
(51) Int. Cl.: A61K 38/43, A61K 9/00

(54) **ALTERING ANIMAL WASTE COMPOSITION**
ÄNDERUNG DER ZUSAMMENSETZUNG TIERISCHER EXKREMENTE
MODIFICATION DE LA COMPOSITION DES DÉJECTIONS ANIMALES

(30) Priority: 02.10.2003 NZ 52864803
(43) Date of publication of application: 02.08.2006
(60) Divisional application: 16152835.1
(73) Proprietor: AgResearch Limited, Hamilton 2020 (NZ)
(72) Inventor: LEDGARD, Stewart Francis, Hamilton, 2001 (NZ)
(74) Representative: Evens, Paul Jonathan
(86) International application number: PCT/NZ2004/000241
(87) International publication number: WO 2005/030247

(56) References cited:
- EP-A- 0 119 494
- WO-A-97/11705
- WO-A-98/42323
- WO-A1-02/19809
- US-A- 4 517 003
- US-A- 4 517 004
- US-A- 4 530 714
- ZERULLA, W. ET AL.: "3,4-dimethylpyrazole phosphate (DMPP)- a new nitrification inhibitor for agriculture and horticulture" BIOL. FERTIL. SOILS, vol. 34, 2001, pages 79-84, XP002437575
- GILL, J.S. ET AL.: "Efficiency of N-(n-butyl) thiophosphoric triamide in retarding hydrolysis of urea and ammonia volatilization losses in a flooded sandy loam soil amended with organic materials" NUTRIENT CYCLING IN AGROECOSYSTEMS, vol. 53, 1999, pages 203-207, XP002437576
- BUNDY, L.G.: "Soil and applied Nitrogen" UNIVERSITY OF WISCONSIN SYSTEM BOARD OF REGENTS AND UNIVERSITY OF WISCONSIN-EXTENSION, 1998, pages 1-6, XP002437577
- VAREL, V.C. ET AL.: "Conservation of nitrogen in cattle feedlot waste with urease inhibitors" J. ANIM. SCI., vol. 77, 1997, pages 1162-1168, XP002437578
- "Full public report on N-(n-butyl) thiophosphoric triamide" NATIONAL INDUSTRIAL CHEMICAL NOTIFICATION AND ASSESSMENT SCHEME, January 1997 (1997-01), XP002437579
- KALBURTJI, K.L. ET AL.,: "Litter dynamics of low and high tannin sericea lespedeza plants under field conditions" PLANT AND SOIL, vol. 208, 1999, pages 271-281, XP002437580
- TAMARA, E.C. ET AL.: "Tannis in nutrient dynamics of forest ecosystems- a review" PLANT AND SOIL, vol. 256, May 2003 (2003-05), pages 41-66, XP002437581
- HERNES, P.J. ET AL.: "Tannin diagenesis in mangrove leaves from a tropical estuary: A novel molecular approach" GEOCHIMICA ET COSMOCHIMICA ACTA, vol. 65, no. 18, 2001, pages 3109-3122, XP002437582
- LUDDEN P.A. ET AL.: 'Influence of the novel urease inhibitor N-(n-butyl)thiophosphoric triamide on ruminant nitrogen metabolism: I. In vitro urea kinetics and substrate digestion' J. ANIM. SCI. vol. 78, no. 1, January 2000, pages 181 - 187, XP003019086
- LUDDEN P.A. ET AL.: 'Influence of the novel urease inhibitor N-(n-butyl)thiophosphoric triamide on ruminant nitrogen metabolism: II. Ruminant nitrogen metabolism, diet digestibility, and nitrogen balance in lambs' J. ANIM. SCI. vol. 78, no. 1, January 2000, pages 188 - 198, XP003019087
- VAREL V.H. ET AL.: 'Conservation of nitrogen in cattle feedlot waste with urease inhibitors' J. ANIM. SCI. vol. 77, no. 5, May 1999, pages 1162 - 1168, XP002437578
- WHITELAW F.G. ET AL.: 'Urease (EC 3.5.1.5) inhibiton on the sheep rumen and its effect on urea and nitrogen metabolism' BR. J. NUTR. vol. 66, no. 2, September 1991, pages 209 - 225, XP001061505
- BRENT B.E. ET AL.: 'In vitro inhibition of rumen urease with acetohydroxamic acid' J. ANIM. SCI. vol. 32, no. 4, April 1971, pages 794 - 798, XP008087742
- MACKIE R.I. ET AL.: 'Biochemical identification and biological origin of key odor components in livestock waste' J. ANIM. SCI. vol. 76, no. 5, May 1998, pages 1331 - 1342, XP003019088

## Description

### TECHNICAL FIELD

This invention relates to a method of altering the composition of animal waste.

### BACKGROUND ART

One of the major environmental issues surrounding intensive farming situations is the release of nitrogen containing compounds into the environment.

Nitrogen is an important component in pastures for the growth and health of plants and soil microorganisms. However, the decay of organic matter such as from plant and animal wastes adds nitrogen to the soil largely in organic forms which plants are unable to use. Microorganisms present in the soil have the ability to convert the organic forms of nitrogen to inorganic forms such as nitrate which can then be taken up by plant roots to generate new organic matter. This conversion of nitrogen is termed The Nitrogen Cycle.

Organic and inorganic forms of nitrogen may also be added to the soil through the addition of fertilisers to pasture.

Nitrogen containing compounds are a significant component in animal waste, particularly urine. Intensive farming practices such as dairying release a large quantity of urine-derived nitrogen to the soil, largely in the form of urea.

Large amounts of nitrogen are however lost from the soil through a number of mechanisms, affecting the productivity of pasture and leading to environmental concerns.

Such mechanisms include denitrification, whereby soil bacteria convert nitrogen containing compounds in the soil to atmospheric nitrogen including nitrous oxide (a greenhouse gas); volatilisation, wherein ammonium salts can volatilise into ammonia which also joins the atmosphere; runoff, which carries nitrogen from the soil into rivers and streams; and leaching, wherein nitrates move through the soil into groundwater.

Urea, applied to the soil either as fertiliser or animal waste, is typically transformed rapidly into ammonium salts and then into nitrate by soil bacteria. Nitrate is highly mobile and moves easily with water through the soil, affecting the quality of groundwater, lakes and streams.

In attempts to minimise the loss of nitrogen from the soil, a number of different methods have been developed.

These methods include feeding of low protein forages to animals to decrease the amount of nitrogen cycling, and the use of feed pads to collect and evenly spread excreta.

Other research groups have been developing new fertiliser compositions containing urease or nitrification inhibitors to slow the conversion of urea to ammonium salts, or from ammonium salts to nitrate respectively, enhancing the residence time of nitrogen containing compounds in the soil and thus reducing the amount of nitrogen lost to the atmosphere or leached from the soil as nitrate.

Published research has also shown reduced nitrate leaching and nitrous oxide emissions from DCD added to cow urine (Klein, C. A. M. de; Logtestijn, R. S. P. van 1994. Denitrification and N2O emission from urine-affected grassland soil. Plant and Soil 163: 235-241; and Di, H.J.; Cameron, K.C. 2002. The use of a nitrification inhibitor, dicyandiamide (DCD), to decrease nitrate leaching and nitrous oxide emissions in a simulated grazed and irrigated grassland. Soil Use and Management 18: 395-403.)

Nitrification inhibitors may be applied directly to soil to reduce nitrification reactions. In grazed pastures, when an animal urinates on the treated ground, the inhibitors slow the conversion of ammonium to nitrate in the urine soaked patch.

However, the application of inhibitors over large areas of ground is problematic. It is difficult to ensure complete or sufficient contact with nitrogen sources prone to high nitrate production (e.g. surface soil nitrogen after cultivation or urine-affected zones in soil). Further, the inhibitors may break down in the environment before being of use, or leach through to ground water.

WO 02/19809 describes a dispensing device located about the tail of a dairy cow and configured to release urease and/or nitrification inhibitors as the cow raises its tail whilst urinating.

WO 02/19809 overcomes some of the practical difficulties in delivering inhibitors into the urine stream and therefore to the zone of soil affected by the urine. However, WO 02/19809 in turn presents practical difficulties of its own as such devices requiring regular checking and maintenance to ensure they contain sufficient inhibitors and have not been knocked or rubbed off, or become fouled or otherwise blocked.

Methods and compositions for the sustained release of active agents in to the bladder have previously been described, particularly in the treatment of bladder cancer in humans.

US 6,306,422 describes the use of urease inhibitors contained within a pH-sensitive polymer matrix which can be coated onto objects such as catheters.

A common problem with catheterised patients is the infection of the urinary tract with urease-producing bacteria. The conversion of urea to ammonium by the bacteria results in an increase in pH of the urine, which in turn causes the pH-sensitive polymer to swell and release antibiotics or urease inhibitors to treat the infection.

In most situations however, ureases are not present in the bladder of an animal and thus the inhibitors remain trapped within the polymer matrix. Accordingly, this composition is not particularly useful for affecting the conversion of urea to ammonium in the absence of infection.

US 6,524,608 describes compositions that have a lower specific gravity than urine, which when infused into the bladder effectively float and are not expelled along with the urine. Instead, the composition is slowly bio-eroded to provide the sustained release of drugs.

Other devices such as that described by US 6,171,298 utilise collapsible balloons placed within the bladder, which can release substances over time.

However, the compositions and devices of the prior art are concerned with the delivery of active substances internally to treat a range of medical conditions, and are not directed to using the urine as a delivery mechanism for active substances to the external environment.

US 4 517 004 relates primarily to arylphosphorictriamide and acryl phosphorodiamide urease inhibitors, and to urease inhibited urea based fertilizer compositions.

No admission is made that any reference constitutes prior art. The discussion of the references states what their authors assert, and the applicants reserve the right to challenge the accuracy and pertinency of the cited documents. It will be clearly understood that, although a number of prior art publications are referred to herein, this reference does not constitute an admission that any of these documents form part of the common general knowledge in the art, in New Zealand or in any other country.

It is acknowledged that the term 'comprise' may, under varying jurisdictions, be attributed with either an exclusive or an inclusive meaning. For the purpose of this specification, and unless otherwise noted, the term 'comprise' shall have an inclusive meaning - i.e. that it will be taken to mean an inclusion of not only the listed components it directly references, but also other non-specified components or elements. This rationale will also be used when the term 'comprised' or 'comprising' is used in relation to one or more steps in a method or process.

It is an object of the present invention to address the foregoing problems or at least to provide the public with a useful choice.

Further aspects and advantages of the present invention will become apparent from the ensuing description which is given by way of example only.

### DISCLOSURE OF INVENTION

According to aspects of the present invention there is provided a method of altering the composition of animal waste and a delivery device for altering the composition of animal waste as defined in the appendant independent claims, to which reference should now be made.

The term "animal waste" should be taken to mean any waste products excreted by the animal such as faeces and urine.

In preferred embodiments of the present invention the animal waste is urine. For ease of reference throughout this specification, the term "animal waste" will be referred to as urine. However, this should not be seen as a limitation on the present invention in any way.

In preferred embodiments of the present invention the animal will be a farm animal, and more preferably a dairy animal such as a cow. Once again, this should not be seen as a limitation as it is anticipated the present invention could be used on other intensively farmed animals such as beef cattle, sheep, goats and so forth, as well as other animal species.

It is desirable to use inhibitors that can be used in small amounts, and which are non-toxic to the animal. The amount of inhibitor administered will be dependent upon a number of factors, including the choice of inhibitor, as well as the body weight of the animal. It is anticipated effective amounts for each inhibitor would be readily discernable to a skilled addressee.

Nitrification inhibitors may be used, alone or in combination with urease inhibitors. For example, a nitrification inhibitor suitable is 3,4-dimethylpyrazole phosphate (DMPP), which has been shown to inhibit nitrification of fertiliser N when applied at relatively low rates and is non-toxic.

Another nitrification inhibitor which may find use is dicyandiamide (DCD).

A number of urease inhibitors are known in the art which may find use in the present invention. For example, N-(n-butyl) thiophosphoric triamide (NBPT) is used commercially under the trade name Agrotain™ and has been used with urea fertiliser to reduce ammonia volatilisation.

Acetohydroxamic acid has been used to control nitrogen metabolism in animals. Further, it should be understood that other substitutes or alternative urease inhibitors might also be used in the present invention.

The invention may optionally also include delivery of one or more other beneficial compounds which function to improve or condition microorganisms already present in the soil, allowing them to better bind the excess nitrogen in urine excreted by animals.

For example, tannins or other complex carbon sources can be used to enhance the immobilisation of nitrogen into soil organic nitrogen, for a more sustained release, a greater livelihood of overall plant recovery and reducing the high inorganic component prone to loss.

For ease of reference only, the term "nitrification inhibitor" will now be referred to simply as a 'nitrogen process modifier'.

The term "nitrogen containing compound" should be taken to mean any compound which contains nitrogen as part of its chemical structure.

In preferred embodiments the nitrogen containing compounds include those found in urine, or produced during the conversion of urine such as urea, ammonium, ammonia and nitrate as well as nitrogen gases including nitrous oxides, and soluble organic forms.

Delivering the nitrogen process modifiers to the environment via animal waste means no or little delay occurs in modifying the conversion of nitrogen containing compounds. Further, this ensures the nitrogen process modifiers are only delivered to the portion of pasture/soil on which the cow urinates, eliminating the need to apply the modifiers over a large area of pasture.

Further, introducing the nitrogen process modifiers internally to the animal eliminates the need for attaching devices to the exterior of the animal, which require regular maintenance to ensure they contain sufficient modifiers, have not been damaged, or have not become fouled or otherwise blocked.

The nitrogen process modifiers (modifiers) of the present invention may be introduced to the animal by a number of mechanisms.

In preferred embodiments of the present invention the modifiers may be introduced to the animal through the digestive system of the animal.

When delivering the modifiers via the digestive system, it is essential to ensure that modifiers maintain their activity and are not denatured or inactivated through the passage of the animal digestive system.

In some embodiments, the bolus and/or modifiers may be encapsulated to provide protection against denaturation and subsequent absorption in the rumen or intestine.

For example, the delivery device may be in the form of a sustained slow release device administered orally to an animal such as a ruminal bolus or a bolus containing a rumen-stable delivery system which preferably includes an encapsulation system to deliver the modifiers post-ruminally.

Such encapsulation systems are well known in the art, including but not limited to polymeric pH-dependent rumen-stable delivery systems, encapsulation by pH-sensitive polymeric coatings or other rumen-stable delivery systems such as heat or chemical treatment, low solubility analogues or lipid-based formulations. Wu S.H.W. and Papas A (1997 - Rumen-stable delivery systems. Advanced Drug Delivery Reviews 28: 323-334) describes a number of such delivery systems.

As understood by a person skilled in the art, a bolus is typically in the form of an elongate cylinder designed to slowly dissolve in the rumen of an animal.

One advantage of having sustained released devices is that the amount of modifiers released can be accurately known. This makes the treatment and the analysis of the effects of the treatment of the animal much more precise than previously.

The bolus will preferably be comprised of a solid matrix, coated with an impervious material having an opening through which the modifiers can be released, or a bio-erodeable coating which releases the modifiers as they are exposed.

A wide variety of boluses and other delivery mechanisms are well known in the art and it is anticipated these technologies could readily be adapted by a skilled addressee for use in the present invention.

In other preferred embodiments the modifiers may be introduced to the animal from a slow release device inserted into an animal's bladder by a farmer or veterinarian.

Preferably, the delivery device will comprise a solid, preformed stable matrix.

For example, the delivery device may be made of silicone matrix which is impregnated with the appropriate modifiers.

In one preferred embodiment the introduction of the modifiers may occur by way of diffusion of those substances from the delivery device over a period of time.

The modifiers may alternatively be delivered through dissolution processes from the matrix or by the placement of appropriate modifiers within the inner core of the delivery device under various delivery systems. Such delivery systems are well known in the art.

In preferred embodiments the delivery device may be bio-erodeable, obviating the necessity for the delivery device to subsequently be removed from the animal after treatment, which would require significant time and effort.

Other preferred embodiments of the present invention may utilise lipid-based delivery vehicles such as liposome-coated compositions, or other slow release matrices known in the art such as bio-erodeable wax coatings, synthetic polymers, cross-linked cellulosic compounds and so forth. For example, in some embodiments weakly acidic or basic co-monomers may be used which slowly release the modifiers during contact with urine.

The sustained release of the modifiers over time to an animal allows the modifiers to be excreted from the animal as a component of the urine, thus directly or indirectly affecting the conversion of ammonium salts to nitrate by soil bacteria.

Other delivery mechanisms may also be used for the application of the modifiers to the animal. These may include incorporating the modifiers in animal feed or by other traditional delivery mechanisms such as drenches or injections.

Other embodiments of the delivery device may utilise an electronic device as powered by an electrochemical cell, to provide sufficient modifiers over the treatment period. Such devices may be inserted into an animal's body cavity and be removed following the treatment period.

While such devices could be inserted into an animal's bladder, these will likely require surgical removal and thus considerable time and labour. Delivery devices may also be located in the rumen of an animal, which may remain in-situ or may be excreted after a period of time.

Methods and compositions for the sustained release of active agents into the bladder have previously before been described, particularly in the treatment of bladder cancer in humans. It is anticipated that a skilled addressee would readily be able to adapt these methods for use in the present invention.

For example, in some embodiments there may be provided a mechanism which can be anchored to a device for later retrieval after the treatment period.

Such a mechanism may be a collapsible slow release membrane balloon. The balloon can be inserted into the bladder through a urethral catheter when in a collapsed form. The balloon can then be injected or infused via the catheter with modifiers. Once the substances have been delivered to the bladder, the balloon can then be withdrawn in its collapsed form through the urethra.

The balloon may deliver the substances through for example, dissolution and therefore will collapse naturally. In other embodiments, the balloon may be punctured to collapse it before it is withdrawn through the urethra.

To enable accurate placement of the collapsible balloon within the bladder, the delivery device may be used to act as an anchor for the balloon component of the delivery device, or alternatively the balloon may effectively "float" in the bladder. One example of such a device is described by US 6,171,298.

Other compositions are known in the art such as those described in US 6,524,608 have a lower specific gravity than urine, and which when infused or inserted into the bladder effectively float and are not expelled along with the urine. Instead, the composition is slowly bio-eroded to provide the sustained release of drugs.

It is anticipated that the use of nitrogen process modifiers will be most effective over a seasonal time period such as from mid-autumn to late-winter, an extended period of approximately three to four months. As such it will be desirable to have a composition or delivery device that slowly released modifiers over this time period.

During this time period, nitrogen is most prone to loss by processes of leaching and/or denitrification/nitrous oxide emission.

However, this should not be seen as a limitation for in other embodiments the modifiers may be delivered over a shorter time period, with a number of treatments administered to the animal as required.

The nitrification inhibitor may be included in a composition including material in the form of biodegradable and/or bio-erodeable matrices known in the art, such as lipid-based coatings, wax coatings, polymer matrices.

As material breaks down in the environment, the modifiers are released and slow or alter the conversion of nitrogen containing compounds in the soil.

Inhibiting or modifying the conversion of nitrogen containing compounds allows more time for the nitrogen excreted in animal waste to be absorbed by plants, before being lost by soil processes such as leaching from the soil as nitrate. This will not only improve soil fertility, but also reduce the level of nitrates entering groundwater and affecting both water quality and drinking water supplies.

It will also likely reduce the levels of nitrous oxide released to the environment and thus reducing levels of greenhouse gases emitted from intensive farming operations.

Having a delivery device which bio-erodes in the bladder allows only a certain amount of modifier to be released at any one time, dependent on the volume of urine excreted by the animal and as such reduces the wastage of modifiers.

The present invention thus encompasses a range of different mechanisms by which nitrogen process modifiers could be administered to animals to achieve their incorporation and excretion in excreta, particularly urine.

Further advantages from this approach include;
1. Targeting the major source of nitrogen loss from pastoral systems (i.e. urine-nitrogen), thereby reducing the amounts needed relative to that for broadcast application over the whole land area
2. Intimate mixing in urine on deposition to soil. This ensures better contact with the urine and more effective modification of nitrogen processes.
3. Most effective timing of deposition, with return in the urine as it is excreted. Additionally, the period of administration to animals can be targeted for greatest reduction in nitrogen losses e.g. from mid-autumn to mid- or late-winter for decreased nitrate leaching.
4. Ability to be administered by the farmer/manager rather than needing a contractor.
5. Potential for a single administration using a slow-release mechanism (e.g. rumen bolus) to achieve effective modification of nitrogen processes over the period of greatest risk of nitrogen losses.

### BRIEF DESCRIPTION OF DRAWINGS

Further aspects of the present invention will become apparent from the ensuing description which is given by way of example only and with reference to the accompanying drawings in which:

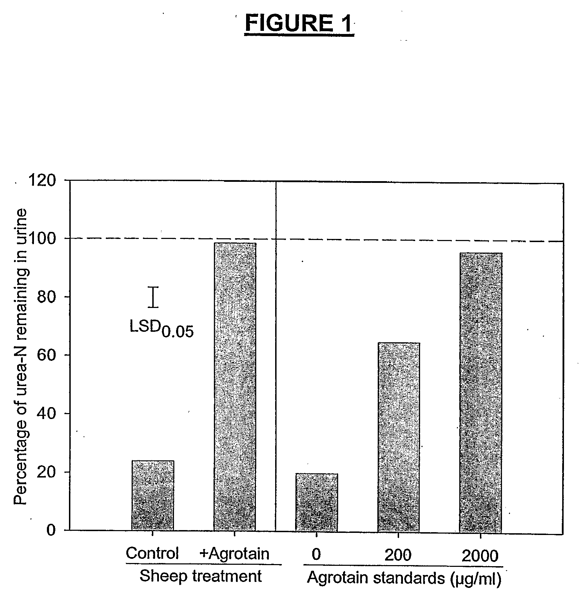

- Figure 1: shows the inhibition of urea hydrolysis in urine from Agrotain-treated sheep (via abomasum infusion) compared to Control (untreated) sheep (average for days 1-3 infusion period). Collected urine had urease added and incubated at 20°C for 165 minutes. Data are compared to that for urine from untreated sheep incubated with urease and different levels of added Agrotain;

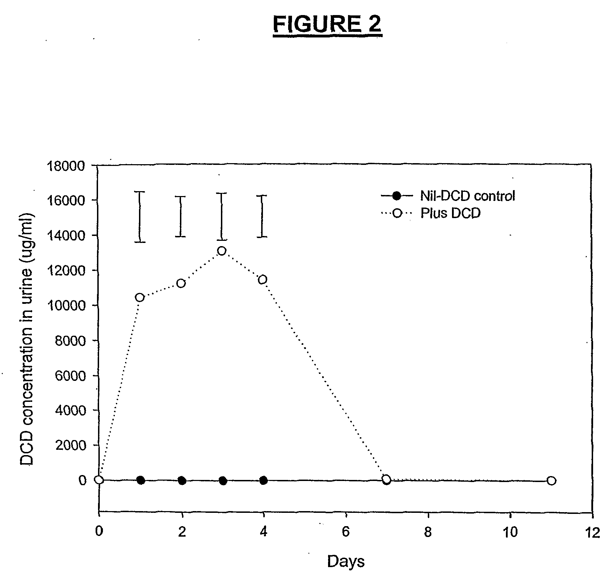

- Figure 2: shows an abomasum infusion study: Temporal changes in DCD concentration in urine (µg/ml) voided from DCD-treated and Control (untreated) sheep covering 1-day pre-treatment, a 4-day infusion period, and 7 days after ceasing infusion. Each data point represents the mean of 11 sheep over 3 experimental periods. Bars are standard errors for +DCD treatment, and

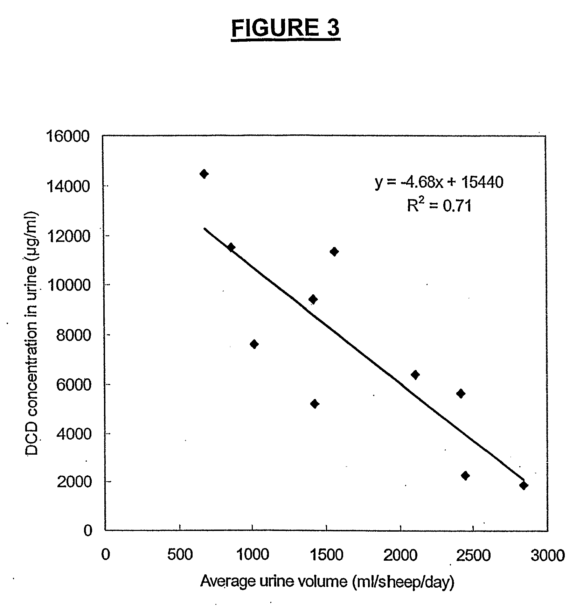

- Figure 3: shows the relationship between the DCD concentration in urine samples from DCD-treated sheep and the average daily volume of urine voided by the sheep, and

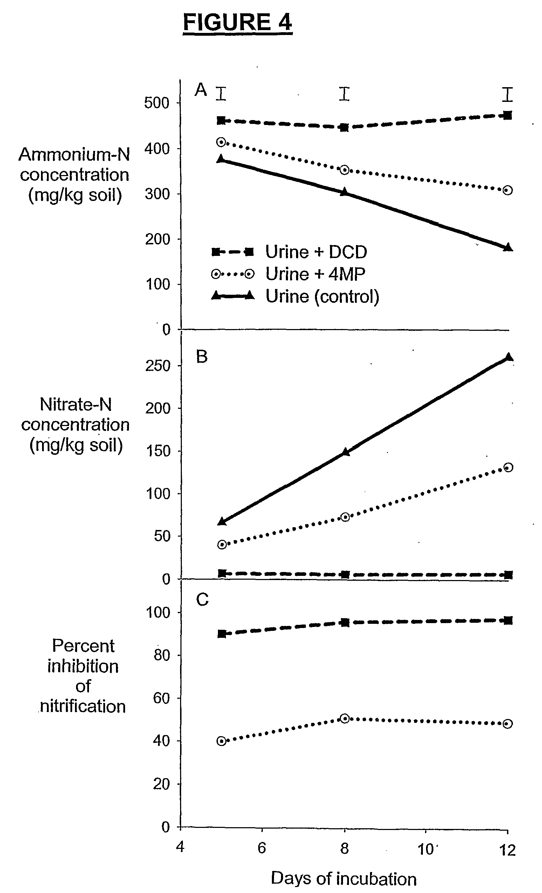

- Figure 4: shows the changes in ammonium (A) and nitrate (B) concentrations and inhibition of nitrification (C) after incubation of soil treated with urine from sheep that had abomasal infusion of water (control), DCD or 4MP. Data are means for urine samples from days 1-4 of infusions over 3 time periods. Error bars for A represent LSD (P<0.05) values. Nitrate data are geometric means and the average Least Significant Ratio (P<0.05) for treatment comparisons is 1.2 for each incubation time. All treatment comparisons for A, B and C showed highly significant differences (P<0.001) at all times. and

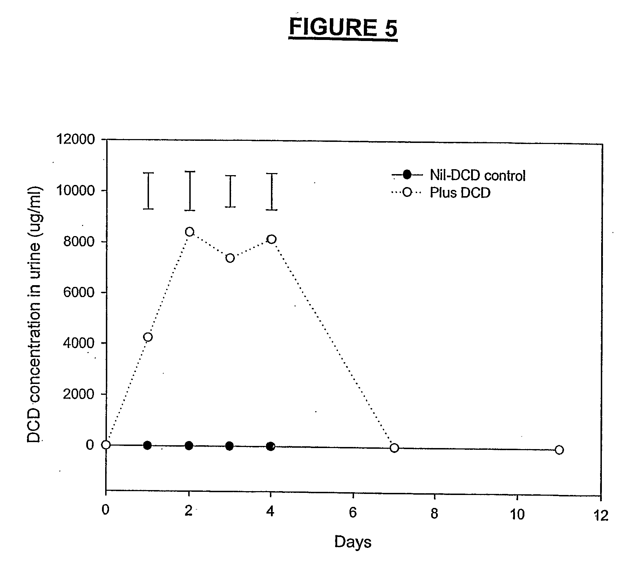

- Figure 5: shows a rumen infusion study: Temporal changes in DCD concentration in urine (µg/ml) voided from DCD-treated and Control (untreated) sheep covering 1-day pre-treatment, a 4-day infusion period, and 7 days after ceasing infusion. Each data point represents the mean of 6 sheep. Bars are standard errors for +DCD treatment, and

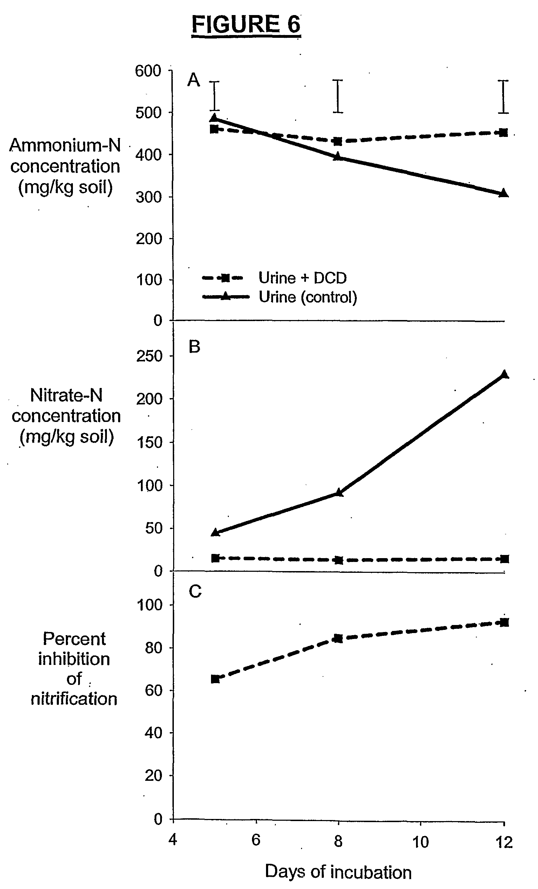

- Figure 6: shows changes in ammonium (A) and nitrate (B) concentrations and inhibition of nitrification (C) after incubation of soil treated with urine from sheep that had ruminal infusion of water (control) or DCD. Data are means for urine samples from days 2-4 of infusions. Error bars for A represent LSD (P<0.05) values. Nitrate data are geometric means and the average Least Significant Ratio (P<0.05) for treatment comparisons is 1.5 for each incubation time. Treatment comparisons for B and C showed highly significant differences (P<0.001) at all times, and

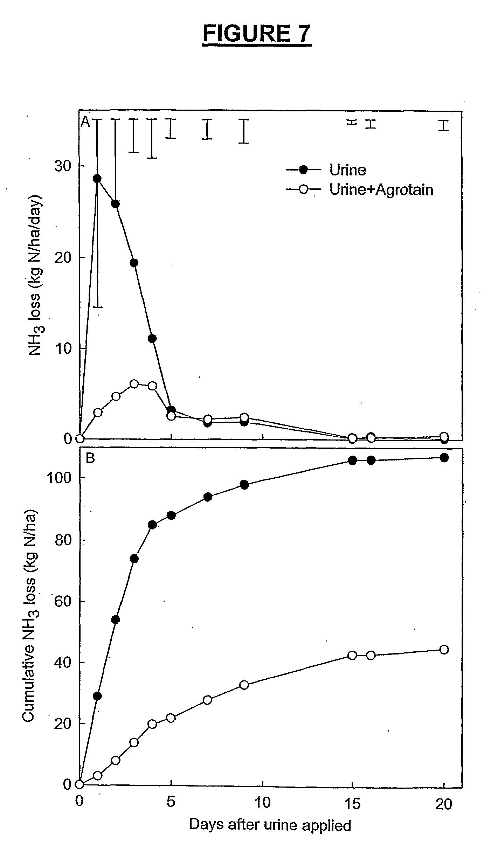

- Figure 7: shows the temporal changes in daily (A) and cumulative (B) ammonia emission from urine with or without added Agrotain. Urine treatments were applied to replicated (n=5) lysimeters with grass/clover pasture on volcanic ash soil. Bars represent LSD (P<0.05) values, and

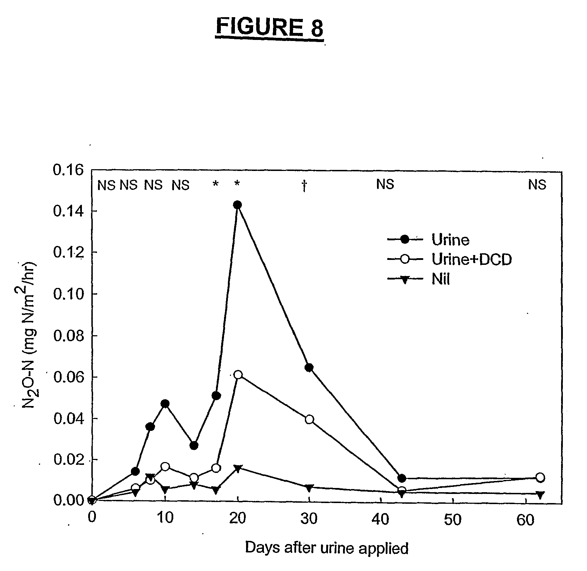

- Figure 8: shows the temporal pattern of nitrous oxide emissions from lysimeters which received control, urine and urine+DCD treatments. Statistical significance is indicated by * (*P*<0.05), † (P<0.1) and NS (not significant), and

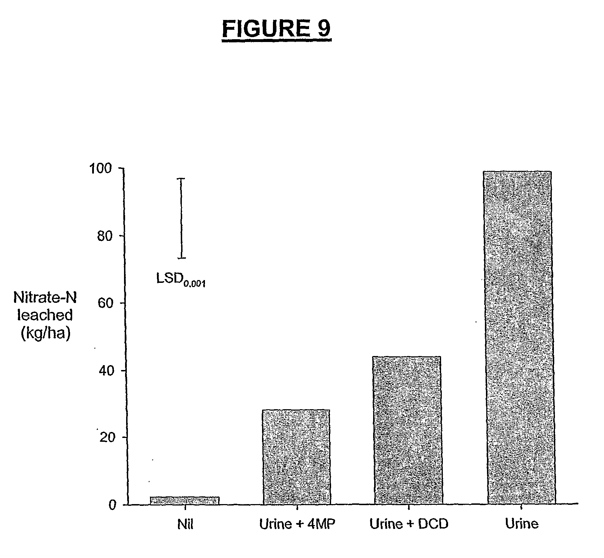

- Figure 9: shows the effect of cow urine and incorporation of the nitrification inhibitors DCD or 4MP in urine on cumulative nitrate leaching over 104 days after application to lysimeters containing grass/clover.

### BEST MODES FOR CARRYING OUT THE INVENTION

As defined above, in its primary aspect, the present invention is directed to a novel method of altering the composition of animal waste by introducing to an animal a nitrification inhibitor that affects the conversion of nitrogen containing compounds in animal waste, once the waste has been excreted from the animal.

The invention is based upon the inventor's investigation into the delivery of nitrogen process modifiers and the modification of nitrogen containing compounds in animal waste.

One preferred embodiment of the present invention consists of a bolus administered orally to grazing animals with targeted delivery to the animal waste, and urine in particular. In preferred embodiments the bolus is in the form of a slow release bolus which is inserted into the rumen and which releases nitrogen transformation modifiers over a sustained period of up to four months. The bolus may additionally include a rumen-stable delivery system to deliver the modifiers post-ruminally.

A generic composition of the bolus is as follows,
i) a core comprising a substantially homogeneous mixture of:
   a) a water insoluble physiologically acceptable binder comprising wax, fat, oil, fatty acid, fatty acid ester, fatty acid amide, fatty acid alcohol or the like organic compound having a melting point above 50°C:
   b) a physiologically acceptable solubilising agent, such as polyethylene glycol stearate or the sodium salt of a long-chain fatty acid,
   c) at least one nitrogen transformation modifier,
   d) where required, a physiologically acceptable inert densifier of sufficient density and in sufficient quantities to give the bolus a minimum density of 1.5g/cm³; and
ii) a coating of a physiologically acceptable material over substantially all of the surface of the core but leaving exposed a core portion whereby in use liquid in the rumen will dissolve said core allowing release of the nitrogen transformation modifier into the rumen.

One preferred embodiment of the bolus may include a nitrogen transformation modifier in the form of nitrification inhibitor such as DMPP or DCD up to 100g with a binding agent such as glycerol monostearate.

The size of the bolus and the amount of modifier required will be dependent on a number of factors, including the identity and concentration of the inhibitor the body weight of the animal and the desired length of treatment. Such factors would be readily discernable to a skilled addressee.

In another preferred embodiment of the present invention the nitrogen process modifiers may be delivered from a slow release composition inserted into the bladder of animals. In these embodiments, a nitrogen inhibitor such as DMPP or DCD is incorporated with a minimal amount of a suitable carrier into a structure such as a flexible filamentous extrusion which is inserted into the bladder using a catheter.

To retain the composition in the bladder, the composition preferably has a specific gravity less than or equal to that of urine, which is normally about 1.005gm/ml to 1.033gm/ml at 25°C. This allows the delivery device to be neutrally buoyant or float in the urine of the bladder minimising the blockage of the urethra.

The composition could contain multiple constituents, a nitrification inhibitor such as DMPP, a urease inhibitor such as NBPT and/or an N-transformation modifier such as a tannin.

### EXAMPLE ONE

### Sheep study on abomasal infusion of inhibitors

A detailed sheep infusion study showed that a significant proportion of inhibitors added into the abomasum were excreted in urine and were effective in inhibiting transformations of urine-N in soil.

Three inhibitors, namely Agrotain (which contains the urease inhibitor NBPT which use on its own is not according to the claims of the present invention), DCD and 4MP (4 methylpyrazole), were evaluated in a replicated cross-over experiment.

Twelve young wether sheep (approximately 35 kg live-weight) had surgery to insert catheters into their rumen and abomasum, and were left for several weeks to recover and to check for normal appetite, growth and bodily function. Sheep were then transferred indoors to metabolism crates. The experiment was carried out in specialist facilities under veterinary supervision and with animal ethics approval.

Sheep were weighed and randomly allocated to a treatment group (3 replicate sheep in each) balanced for liveweight. There were four infusion treatments: water infusion (Control), Agrotain at 80 g/day (Agrotain group), DCD at 15 g/day (DCD group) and 4MP at 1 g/day (4MP group). The inhibitor compounds were dissolved in distilled water before infusion. The experiment was repeated over 3 consecutive time-frames to give 3 replicated experimental periods, with details as follows.

During the first period, animals received the treatments in solution as continuous abomasum infusions for a period of 5 days. An exception was the Agrotain group, where infusion of Agrotain ceased after day 3 due to reduced feed intake by animals and was replaced with water for the remaining 2 days.

Following the infusion period, animals had a 10 day non-treatment clearance/rest period, before being allocated to one of three treatments (4 sheep per treatment): water infusion (Control), DCD at 15 g/day and 4MP at 1 g/day. Infusion of treatments continued for 4 days, after which there was an 11 day non-treatment clearance/rest period before repeating this phase. Sheep allocation to treatments changed over each period in a statistical cross-over design (defined by statisticians) so that 11 sheep had been subjected to each of 3 treatments (control, DCD and 4MP), and so that within- and between-sheep variability could be evaluated.

During each infusion period the sheep were housed in metabolism crates to allow daily collection of urine and faeces samples. Single urination event samples were also collected from each sheep every day and were frozen for subsequent analysis of inhibitor content and for inhibitory activity in soil. Initial laboratory studies showed that freezing urine at -20°C had no effect on the presence or activity of Agrotain or DCD.

Urea hydrolysis (i.e. transformation of urea to ammonium) was determined in excreted urine samples from the control and Agrotain treatment sheep. This involved addition of urease, incubation at 20°C for 165 minutes, followed by colourimetric determination of urea concentration. Replicated standard samples with urine, urease and different levels of Agrotain addition were analysed for comparison.

Results from this evaluation showed significantly less urea hydrolysis in urine samples from Agrotain-treated sheep than from control sheep (Figure 1), indicating effective inhibition of urea hydrolysis by Agrotain. This indicates that NBPT had been absorbed through the abomasum wall and transferred via the blood-stream to the bladder without being metabolised to an ineffective state. The standard samples highlighted the effect of increasing rate of Agrotain addition on inhibiting urea hydrolysis (Figure 1) and indicate that a relatively high rate of NBPT from Agrotain was excreted in urine from Agrotain-treated sheep.

Presence of DCD in urine samples was determined using high-performance liquid chromotography. There was a highly significant increase (P<0.001) in the DCD concentration in urine from all DCD-treated sheep at all days during the infusion period (Figure 2). This showed that high levels of DCD were absorbed in the abomasum and excreted in urine in an unaltered form. There was some variability between sheep in DCD concentrations in urine, but this reflected differences in urination volumes (Figure 3) so that there was much less difference in the amount of DCD excreted per urination. Concentrations of DCD in urine declined to negligible levels within 7 days after ceasing infusion (Figure 2).

Inhibition of nitrification (i.e. the conversion of ammonium to nitrate) of urine/urea-N in soil was clearly evident in urine from all sheep treated with DCD or 4MP at all samplings (Figure 4).

This was determined by firstly measuring the urea concentration in urine samples and adjusting the urine volume (or in a few samples with low concentrations, urea was added) so that the same rate of urea-N was added to soil (100 g) in plastic bags. The soil was a Horotiu silt loam derived from volcanic ash taken from a pastoral site which had been ungrazed for over 6 months so that no residual animal urine was present.

The urine-treated soil samples were incubated at 20°C and sub-samples were removed at regular intervals over time for analysis of ammonium and nitrate in 2M KCI extracts using standard methods. The level of inhibition of nitrification was evident from less nitrate formation (Figure 4B; P<0.001 at all incubation times) and higher ammonium levels (Figure 4A; P<0.001 at all incubation times). Inhibition of nitrification was higher for DCD than for 4MP (P<0.01), with an average reduction in nitrate production relative to the control of 96 and 51%, respectively (Figure 4C). This difference may have been influenced by the rate of inhibitor used.

There was no adverse effect of DCD or 4MP infusion on sheep in terms of live-weight changes, body condition, and general bodily function, which was to be expected since both compounds are recognised as non-toxic substances. However, Agrotain did affect the sheep as evident by greatly reduced feed-intake and at day 4 after starting the first infusion period, this treatment stopped. While NBPT should have had no effect on animals at the levels used, it is possible that other compounds present in the Agrotain had a negative effect on the sheep.

### EXAMPLE TWO

### Sheep study on ruminal infusion of inhibitors

A detailed sheep ruminal infusion study showed that DCD added into the rumen was excreted in urine and was effective in inhibiting nitrification of urine-N in soil. However, the level of excretion of DCD in urine was less than that in the abomasal infusion study, suggesting some metabolism of DCD in the rumen and/or greater excretion of DCD in dung.

The sheep from the abomasal infusion study had a two week clearance/rest period, and were then used for a second study, where the infusion apparatus was connected to the rumen catheters. The 12 sheep were randomly allocated to one of 2 treatments; water infusion (Control) or DCD at 15 g/day (DCD group). Experimental methodology was the same as for the abomasal infusion study in that there was a 4 day period of continuous infusion followed by an 11 day non-treatment clearance/rest period.

Presence of DCD in urine samples was determined using high-performance liquid chromotography. This indicated that high levels of DCD were absorbed and excreted in urine in an unaltered form in all sheep and was present at all samplings over the infusion period (Figure 5). However, concentrations of DCD in urine were lower at day 1 of infusion than days 2-4, as expected because of time for rumen processing. Levels declined after ceasing infusion and were negligible within 7 days (Figure 5).

Inhibition of nitrification of urine/urea-N in soil was clearly evident in urine from all sheep treated with DCD compared to that from untreated sheep at all samplings (Figure 6). Nitrate formation was greatly diminished In the DCD treatment (Figure 6B; P<0.001 at all incubation times), with an average reduction in nitrate production of 81%.

### EXAMPLE THREE

### Lysimeter study on the effect of inhibitors on N losses

A detailed lysimeter study showed that inhibitors added to urine were effective in reducing N losses, with Agrotain reducing ammonia emissions and DCD and 4MP reducing nitrate leaching and nitrous oxide (a greenhouse gas) emissions.

Undisturbed soil lysimeters (30 cm diameter x 50 cm depth) were collected from a pastoral site near Lake Taupo which had been under a cutting regime with no grazing animals for over 18 months. The soil was a Taupo sandy loam (yellow brown pumice or allophanic soil). A gap (c. 1 cm) between the side of the soil cores and the PVC lysimeter housing was filled with Vaseline to stop possible edge-flow of water or urine. The lysimeters were transferred to a lysimeter facility (near Hamilton, New Zealand) where they were buried in soil so that the soil surface in the lysimeter was at ground level. However, the lysimeter casing extended about 4 cm above the soil surface to stop any lateral surface loss.

There were five replicate lysimeters with the following treatments included: a control, urine, urine + DCD, and urine + 4MP. Urine was collected from Jersey dairy cows, stored at 4°C and applied to the lysimeters within 4 days. The urine solution was applied at 10 L/m² giving the equivalent of 800 kg N/ha (similar to that for a cow urine patch). For the DCD and 4MP treatments, the inhibitor compounds were dissolved in the urine prior to application to represent urine excreted from inhibitor-administered animals. Inputs in these treatments were equivalent to 1.5 g DCD/m² and 0.1 g 4MP/m².

Lysimeters were left to stabilise for several weeks after installation in the facility. Treatments were applied in May 2004. Pasture on the lysimeters was trimmed prior to treatment application and was regularly cut at heights and intervals similar to that under dairy cow grazing.

In addition to the main study, extra shallow lysimers (20 cm depth) were collected and treated in the same way as the main lysimeters. There were 5 replicates of the same treatments. These were used for measurement of ammonia loss from the urine and urine+Agrotain treatments.

Ammonia loss measurement involved placement of a clear Perspex cover over each lysimeter which had holes on one side and a hose on the other side connected to a Dreschel bottle containing acid and to a vacuum source. During a sequence of 24 hour periods, air was drawn across the top of each lysimeter and passed through the acid to absorb ammonia for subsequent analysis. Ammonia losses from urine were high during the first 24 hours after application and declined rapidly over the following 4 days (Figure 7A). Addition of Agrotain to urine greatly reduced ammonia loss during this period and halved cumulative emissions over 20 days from 13% to 6% of the urine-N applied (Figure 7B).

Nitrous oxide (N₂O) emission from the main lysimeters was measured using a standard closed chamber method. Chambers were placed on the lysimeters at regular intervals during the study for periods of one hour at around midday. Gas samples were collected at 0, 30 and 60 minutes from the headspace of the chambers for N₂O analysis by gas chromatography. Emissions of N₂O varied over time but were significantly reduced in the urine treatment with DCD added during the 14-30 day period after application (Figure 8). Cumulative N₂O emissions over the measurement period averaged 1.2 and 0.5 kg N/ha for the urine and urine+DCD treatments, giving a 57% reduction from DCD.

Nitrate leaching from lysimeters was measured by collection of leachate draining from the base of each lysimeter. This drainage water was collected at regular intervals and analysed for nitrate concentration. Cumulative nitrate leaching was highest from the urine treatment and was reduced by 56 or 71% in treatments where DCD or 4MP was added to urine, respectively (Figure 9).

Results from these experiments have shown the potential for nitrogen process modifiers to be delivered to animals such that they are excreted in urine and reduce associated nitrogen losses into the environment.

The abomasal infusion study showed that three different nitrogen process modifiers were absorbed via the abomasum and excreted in urine, resulting in significant changes in transformations of urine-nitrogen. The Agrotain-treated sheep produced urine with slower urea hydrolysis which can reduce ammonia loss when deposited on soil. The DCD and 4MP treated sheep produced urine with slower nitrification, which can reduce N losses by leaching and nitrous oxide emission. In the case of the DCD treatment, this was confirmed by analysis of DCD in the urine as well as by large inhibition in rate of nitrification of urine-nitrogen in soil.

The rumen infusion study confirmed that DCD administered into the rumen resulted in its excretion in urine and inhibition of nitrification of urine-nitrogen. Application of urine containing these inhibitors to pastoral soil in the lysimeter experiment showed reduced nitrogen emissions via ammonia volatilisation (from Agrotain) or from nitrate leaching and nitrous oxide emission (from DCD and 4MP).

## Claims

1. A method of altering the composition of animal waste,
**characterised by** the step of
introducing internally to an animal a nitrification inhibitor that can directly or indirectly affect the conversion of nitrogen containing compounds in animal waste once the animal waste has been excreted,

2. A delivery device for altering the composition of animal waste,
**characterised in that**
the delivery device is adapted to deliver internally to an animal a nitrification inhibitor that can directly or indirectly affect the conversion of nitrogen containing compounds in animal waste once the animal waste is excreted from the animal,

3. A delivery device as claimed in claim 2 herein the animal waste is urine.

4. A delivery device as claimed in a claim 2 or claim 3 wherein the nitrification inhibitor is 3,4-dimethylpyrazole phosphate (DMPP).

5. A delivery device as claimed in any one of claims 2 to 4 wherein the nitrification inhibitor is dicyandiamide (DCD).

6. A delivery device as claimed in any one of claims 2 to 5 wherein said delivery device is configured to be administered orally to an animal.

7. A delivery device as claimed in claim 6 wherein the delivery device is a ruminal bolus.

8. A delivery device as claimed in either one of claims 6 or 7, wherein the delivery device is a rumen-stable delivery device.

9. A delivery device as claimed in claim 8 wherein the delivery device includes an encapsulation system.

10. A delivery device as claimed in any one of claims 2 to 5, wherein said delivery device is configured to be inserted into an animal's bladder.

11. A delivery device as claimed in a claim 10 wherein the delivery device has a lower specific gravity than urine.

12. A delivery device as claimed in any one of claims 2 to 11 wherein the nitrification inhibitor is contained within an inner core of the delivery device.

13. A delivery device as claimed in any one of claims 2 to 10 wherein the delivery device is impregnated with the nitrification inhibitor.

14. A delivery device as claimed in any one of claims 2 to 13, wherein the nitrification inhibitor is delivered by diffusion from the delivery device.

15. A delivery device as claimed in any one of claims 2 to 13, wherein the nitrification inhibitor is delivered through dissolution of the delivery device.

16. A delivery device as claimed in any one of claims 2 to 15, wherein the delivery device includes an electrochemical cell.

17. A delivery device as claimed in any one of claims 2 to 16 further including a urease inhibitor.

18. A delivery device as claimed in claim 17 wherein the urease inhibitor is N-(n-butyl) thiophosphoric triamide (NBPT).

19. The use of a nitrification inhibitor in the manufacture of a delivery device as claimed in any one of claims 2 to 18.

20. A method as claimed in claim 1 wherein the nitrification inhibitor is introduced by way of a delivery device as defined in any one of claims 2 to 18.

## Patentansprüche

1. Verfahren zur Änderung der Zusammensetzung tierischer Nebenprodukte,
**gekennzeichnet durch** den folgenden Schritt:
Einführen eines Nitrifikationsinhibitors in ein Tier, der die Umwandlung von stickstoffhaltigen Verbindungen in tierischen Nebenprodukten direkt oder indirekt beeinflussen kann, sobald das tierische Nebenprodukt ausgeschieden wurde.

2. Abgabevorrichtung zur Änderung der Zusammensetzung tierischer Nebenprodukte,
**dadurch gekennzeichnet, dass**
die Abgabevorrichtung angepasst ist, um einen Nitrifikationsinhibitor in einem Tier abzugeben, der die Umwandlung von stickstoffhaltigen Verbindungen in tierischen Nebenprodukten direkt oder indirekt beeinflussen kann, sobald das tierische Nebenprodukt ausgeschieden wurde.

3. Abgabevorrichtung nach Anspruch 2, wobei das tierische Nebenprodukt Urin ist.

4. Abgabevorrichtung nach Anspruch 2 oder Anspruch 3, wobei der Nitrifikationsinhibitor 3,4-Dimethylpyrazolphosphat (DMPP) ist.

5. Abgabevorrichtung nach einem der Ansprüche 2 bis 4, wobei der Nitrifikationsinhibitor Dicyandiamid (DCD) ist.

6. Abgabevorrichtung nach einem der Ansprüche 2 bis 5, wobei die Abgabevorrichtung konfiguriert ist, um einem Tier oral verabreicht zu werden.

7. Abgabevorrichtung nach Anspruch 6, wobei die Abgabevorrichtung ein ruminaler Bolus ist.

8. Abgabevorrichtung nach einem der Ansprüche 6 oder 7, wobei die Abgabevorrichtung eine pansenstabile Abgabevorrichtung ist.

9. Abgabevorrichtung nach Anspruch 8, wobei die Abgabevorrichtung ein Verkapselungssystem einschließt.

10. Abgabevorrichtung nach einem der Ansprüche 2 bis 5, wobei die Abgabevorrichtung konfiguriert ist, um in die Blase eines Tiers eingesetzt zu werden.

11. Abgabevorrichtung nach Anspruch 10, wobei die Abgabevorrichtung ein niedrigeres spezifisches Gewicht hat als Urin.

12. Abgabevorrichtung nach einem der Ansprüche 2 bis 11, wobei der Nitrifikationsinhibitor in einem inneren Kern der Abgabevorrichtung enthalten ist.

13. Abgabevorrichtung nach einem der Ansprüche 2 bis 10, wobei die Abgabevorrichtung mit dem Nitrifikationsinhibitor getränkt ist.

14. Abgabevorrichtung nach einem der Ansprüche 2 bis 13, wobei der Nitrifikationsinhibitor durch Ausbreitung von der Abgabevorrichtung abgegeben wird.

15. Abgabevorrichtung nach einem der Ansprüche 2 bis 13, wobei der Nitrifikationsinhibitor durch Auflösung der Abgabevorrichtung abgegeben wird.

16. Abgabevorrichtung nach einem der Ansprüche 2 bis 15, wobei die Abgabevorrichtung eine elektrochemische Zelle einschließt.

17. Abgabevorrichtung nach einem der Ansprüche 2 bis 16, die ferner einen Ureaseinhibor einschließt.

18. Abgabevorrichtung nach Anspruch 17 wobei der Ureiseinhibitor N-(n-Butyl)-thiophosphortriamid (NBPT) ist.

19. Verwendung eines Nitrifikationsinhibitors bei der Herstellung einer Abgabevorrichtung nach einem der Ansprüche 2 bis 18.

20. Verfahren nach Anspruch 1, wobei der Nitrifikationsinhibitor durch eine Abgabevorrichtung nach einem der Ansprüche 2 bis 18 eingeführt wird.

## Revendications

1. Procédé de modification de la composition des déchets d'origine animale,
**caractérisé par** l'étape
d'introduction à l'intérieur de l'animal un inhibiteur de nitrification susceptible directement ou indirectement d'affecter la conversion de composés contenant de l'azote dans les déchets d'origine animale après excrétion.

2. Dispositif d'administration permettant de modifier la composition des déchets d'origine animale,
**caractérisé en ce que**
le dispositif d'administration permet d'administrer à l'intérieur de l'animal un inhibiteur de nitrification susceptible directement ou indirectement d'affecter la conversion de composés contenant de l'azote dans les déchets d'origine animale après excrétion.

3. Dispositif d'administration selon la revendication 2, où les déchets d'origine animale sont de l'urine.

4. Dispositif d'administration selon la revendication 2 ou la revendication 3, où l'inhibiteur de nitrification est le 3,4-diméthylpyrazole phosphate (DMPP).

5. Dispositif d'administration selon l'une quelconque des revendications 2 à 4, où l'inhibiteur de nitrification est le dicyandiamide (DCD).

6. Dispositif d'administration selon l'une quelconque des revendications 2 à 5, où ledit dispositif d'administration est configuré pour une administration par voie orale à un animal.

7. Dispositif d'administration selon la revendication 6, où le dispositif d'administration est un bolus ruminai.

8. Dispositif d'administration selon l'une ou l'autre des revendications 6 ou 7, où le dispositif d'administration est dispositif d'administration stable dans la panse.

9. Dispositif d'administration selon la revendication 8, où le dispositif d'administration contient un système d'encapsulation.

10. Dispositif d'administration selon l'une quelconque des revendications 2 à 5, où ledit dispositif d'administration est configuré pour une introduction dans la vessie d'un animal.

11. Dispositif d'administration selon la revendication 10, où le dispositif d'administration présente une gravité spécifique inférieure à l'urine.

12. Dispositif d'administration selon l'une quelconque des revendications 2 à 11, où l'inhibiteur de la nitrification est contenu dans un noyau intérieur du dispositif d'administration.

13. Dispositif d'administration selon l'une quelconque des revendications 2 à 10, où le dispositif d'administration est imprégné de l'inhibiteur de nitrification.

14. Dispositif d'administration selon l'une quelconque des revendications 2 à 13, où l'inhibiteur de nitrification est administré par diffusion à partir du dispositif d'administration.

15. Dispositif d'administration selon l'une quelconque des revendications 2 à 13, où l'inhibiteur de nitrification est administré par dissolution du dispositif d'administration.

16. Dispositif d'administration selon l'une quelconque des revendications 2 à 15, où le dispositif d'administration contient une cellule électrochimique.

17. Dispositif d'administration selon l'une quelconque des revendications 2 à 16 comprenant en outre un inhibiteur d'uréase.

18. Dispositif d'administration selon la revendication 17, où l'inhibiteur d'uréase est le triamide N-(n-butyle) thiophosphorique (NBPT).

19. Utilisation d'un inhibiteur de nitrification dans la fabrication d'un dispositif d'administration selon l'une quelconque des revendications 2 à 18.

20. Procédé selon la revendication 1, où l'inhibiteur de nitrification est introduit à l'aide d'un dispositif d'administration selon l'une quelconque des revendications 2 à 18.
